# EUROPEAN PATENT APPLICATION

(11) **EP 4 678 651 A2**
(43) Date of publication of application: **14.01.2026**
(21) Application number: 25189183.4
(22) Date of filing: 11.07.2025
(51) Int. Cl.: C07K 7/06, A61K 47/64, C07K 7/08, C07K 14/435, C07K 14/47

(54) **BRANCHED PEPTIDES WITH ANTIBACTERIAL AND ANTI-INFLAMMATORY ACTIVITY**

(30) Priority: 12.07.2024 IT 202400016213
(71) Applicant: Universita' Degli Studi di Siena, 53100 Siena (IT)
(72) Inventor: FALCIANI, Chiara, 53100 Siena (IT); BRACCI, Luisa, 53100 Siena (IT); PINI, Alessandro, 53100 Siena (IT); TOLLAPI, Eva, 53100 Siena (IT)
(74) Representative: Valenza, Silvia

(57) **Abstract**

The present invention describes a branched peptide of formula (I), the use of the same as an antibacterial, and related pharmaceutical compositions or medical devices.

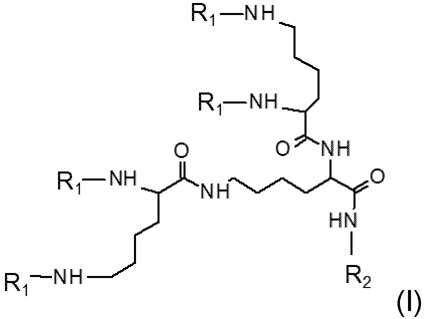

## Description

### FIELD OF THE INVENTION

The present invention refers to the field of peptides with antibacterial and anti-inflammatory activity, and pharmaceutical or cosmetic preparations, or medical devices thereof.

### STATE OF THE ART

The growing phenomenon of antimicrobial resistance (AMR), accompanied by serious health risks, underlines the urgency of intensifying the production of new antibiotics and discovering innovative strategies to fight infectious diseases. Life-threatening infections caused by "superbugs" are becoming increasingly frequent, not only in hospitalized and immunocompromised patients, but also among healthy young people. The World Health Organization (WHO) warns that antimicrobial resistance (AMR) is an increasingly serious threat to global public health and calls for action across all government sectors and society *(https:*//*www.who.int*/*health-topics*/*antimicrobial-resistance).* Bacteria that cause local and minor infections, for example of the urinary tract or oral cavity, now show high rates of resistance in all regions of the world.

Prolonged use of antibiotics contributes to the development and spread of drug-resistant microorganisms. Therefore, new treatment options are urgently needed and, most importantly, every effort should be made to preserve the efficacy of existing antibacterial agents currently used to treat life-threatening infections. All other uses of new antimicrobials, such as post-surgical prophylaxis and treatment of trivial infections, should be addressed with non-life-saving antibiotics. Not only are new treatment options needed, but different classes of molecules should be administered in different settings of local and severe infections to preserve life-saving drugs from resistance.

Peptide antibiotics, such as bacitracin, colistin, and daptomycin, are currently employed in managing infectious diseases, while many others are undergoing evaluation for potential clinical use. Natural antimicrobial peptides (AMPs) are found in all classes of living organisms as part of innate immune responses *(*Diamond G. et al. Curr Pharm Des. 2009*).* They are believed to act as broad-spectrum antibiotics that kill bacteria, viruses, and fungi, and to have immunomodulatory properties as well. AMPs also show a reduced tendency to promote AMR development in pathogenic bacteria.

AMPs are continuously produced by living organisms due to their short half-life and limited killing power *(*Uddin SJ. et al. Front Pharmacol. 2021*).* Researchers have tried many chemical modifications to overcome their ephemerality. One such modification involves synthesizing the active sequence on a branched core, which provides the entire molecule with an increased half-life and enhanced bactericidal activity. Tetrabranched AMPs show promise for fighting MDR infections due to their broad spectrum of activity and their resistance to proteases and peptidases. For example, the peptide M33 was developed in a preclinical setting and showed interesting MIC90 and biofilm eradication properties against a wide panel of bacterial species. M33 also showed immunomodulatory activity, along with *in vivo* activity in cases of sepsis, pneumonia, and bacterial skin infections in mice *(*Cresti L. et al. Sci Rep. 2022*;* Marianantoni G. et al. Pharmaceutics. 2022*).*

The need for new compounds with antibacterial and possibly anti-inflammatory activity is therefore apparent.

### SUMMARY OF THE INVENTION

The present invention addresses the above problem by means of a branched peptide, or pharmaceutically acceptable salts thereof, said peptide having formula (I) 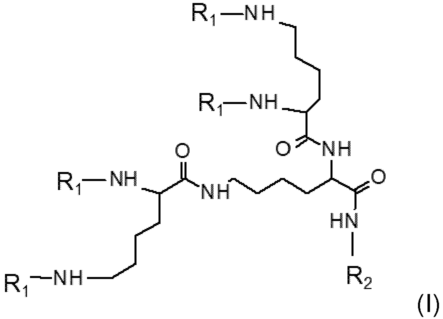 wherein
- R₁: equal or different from each other are peptides having SEQ ID NO: 1-12, or portions thereof, or homologs thereof with at least 80% homology, wherein the amino acids can be of the L series or D series;
- R₂: is Ala (A), beta-Ala (beta-A), PEG, or is absent.

The branched peptides of the invention proved to be effective as antibacterial agents against various strains of different species. Their efficacy was also proved to be significantly higher than that of monomeric analogues, already known in the literature as antibacterial agents. The tetrabranched peptides also demonstrated antibiofilm activity, resistance to proteases, and low cytotoxicity for eukaryotic cells. The peptides also proved to be effective in binding LPS, a bacterial toxin capable of inducing inflammation and worsening of infection clinical picture.

One of the peptides, BAMP2, has demonstrated *in vivo* efficacy in a mouse model of skin infection and has shown promise for reducing infection and inflammation. An object of the present invention is therefore also a branched peptide of formula (I) as defined above for use as a medicament, preferably for use in the treatment of bacterial infections.

The peptides have shown antibacterial efficacy when incorporated into a cream base, at the same active concentrations as in the absence of the cream. Therefore, they are promising when formulated into creams suitable for use as topical antibiotics for skin infections or acne, and as cosmetic preservatives as well.

In one aspect, the present invention therefore relates to a pharmaceutical composition comprising a branched peptide of formula (I) as defined above, and at least one other pharmaceutically acceptable ingredient, said composition preferably being in the form of a cream or other pharmaceutical form suitable for topical administration.

An object of the present invention is also the use of a branched peptide of formula (I) as a preservative in cosmetic compositions.

The peptides have shown antibacterial efficacy when incorporated into a gel-forming material, such as collagen, at the same active concentrations as in the absence of a gel. Therefore, an object of the present invention is also a gel comprising a branched peptide of formula (I), said gel preferably for use in the treatment of wounds (wound-dressings or other wound dressing devices) or bone infections in orthopedics or endodontics (for example, as an antimicrobial coating for prosthetic implants).

The tetrabranched peptides of the invention were shown to be effective against bacterial strains typical of root canal infections. These results confirm that these antimicrobial peptides may also be valid alternatives for dental treatments against infections.

### DETAILED DESCRIPTION OF THE INVENTION

SEQ ID NO: 1-12 are shown below:

| | |
|---|---|
| SEQ ID NO: 1 | TLLKKVLKAAAK (BAMP2) |
| SEQ ID NO: 2 | LLKKVLLAAAK (BAMP2.3) |
| SEQ ID NO: 3 | GIIDIAKKLFESW (BAMP18) |
| SEQ ID NO: 4 | LRAAHRLAIGRR (BAMP23) |
| SEQ ID NO: 5 | KLLKRIKKLL (BAMP35) |
| SEQ ID NO: 6 | KKLLGKNWKLM (BAMP37) |
| SEQ ID NO: 7 | LKKVLKAAAK (BAMP39) |
| SEQ ID NO: 8 | LKLKSIVSW (BAMP41) |
| SEQ ID NO: 9 | INLKKLAKL (BAMP45) |
| SEQ ID NO: 10 | GRFKRFRKKL (BAMP49) |
| SEQ ID NO: 11 | RGLRRLGRKIA (BAMP52) |
| SEQ ID NO: 12 | tllkkvlkaaak (BAMP2D) |

The peptides object of the present invention were synthesized in a tetrabranched form using three lysins as scaffolds to support four (preferably identical copies of) sequences on four amino groups of the lysins. The rationale behind transforming natural peptides into small dendrimers is related to the possibility of increasing their stability to proteases and binding affinity. When compared to monomers thereof, the branched peptides of the invention show a significant increase in resistance to serum proteolytic activity, likely due to their bulkier size, which prevents the peptide from fitting into the protease cleavage site. Multivalency, on the other hand, allows for stronger binding to the bacterial outer membrane, resulting in faster killing, as well as to LPS and LTA, thus producing favorable immunomodulation. Increased activity and resistance allow for lower doses and help overcome toxicity drawbacks.

Preferably, in the branched peptide of formula (I) according to the present invention, R1 residues are equal to each other, i.e., the R1 residue is repeated four times. The amino acids of the R1 sequence may be in the L or D form.

Peptides including the following R1 are to be intended as included in formula (I):
- a portion of the sequences (shortenings)
- and/or at least 80% homology to the sequences indicated above.

Preferably R1 is selected from the group consisting of SEQ ID NO. 1 (BAMP2), SEQ ID NO. 2 (BAMP2.3), SEQ ID NO. 12 (BAMP2D).

Preferably R2 is absent.

A tetrabranched peptide according to the present invention with four identical R1 can be synthesized using the amino acid Fmoc-Lys (Fmoc)-OH to generate the lysine core. In the case of four residues that are present in different pairs, the amino acid Lys with two orthogonal protecting groups, for example, (Fmoc Lys (Dde)-OH), can be used in the first or second step of the core synthesis, so as to differentiate the two different sequences with selective deprotections.

The present invention can be better understood based on the following embodiment examples.

### BRIEF DESCRIPTION OF THE FIGURES

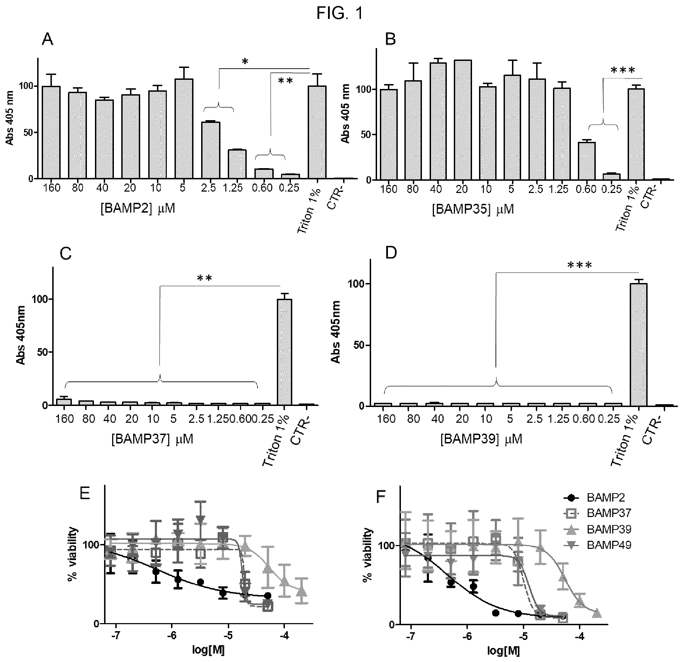

**Fig. 1 A-F** Hemolytic activity of BAMP peptides at 37°C for 2 h. All experiments were repeated at least three times (n=3); statistical analysis was performed using the paired two-tailed *t* test. The asterisk indicates significant differences between columns (*, p < 0.05; **, p < 0.01; ***, p < 0.001). Cytotoxicity at 4 h **(G)** and 24h **(H)**

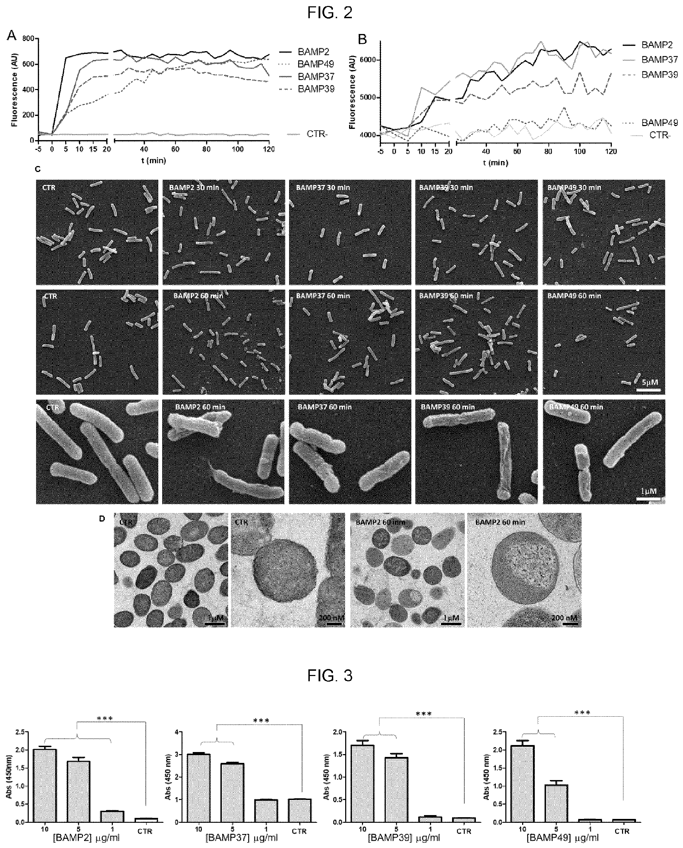

**Fig. 2 A-B** Cytoplasmic membrane permeability of E. coli to BAMP treatment at the MIC and twice the MIC. Fluorescence due to binding of PI (A) and Sytox green (B) fluorescent probes to DNA was measured at excitation and emission wavelengths of 535-617 nm and 480-523 nm, respectively, with a plate reader. Data are expressed as means (±SD) of three independent repeats in triplicate. **(C)** SEM images of E. coli treated with the peptides, a magnified insert is shown in yellow, and **(D)** TEM images of E. coli treated with the BAMP2 peptide, where yellow arrows indicate loss of the outer membrane double layer and vesicles.
**Fig. 3** Binding of BAMP to LPS was tested by ELISA with LPS-biotin and streptavidin-POD. ***p<0.0001 n=12.

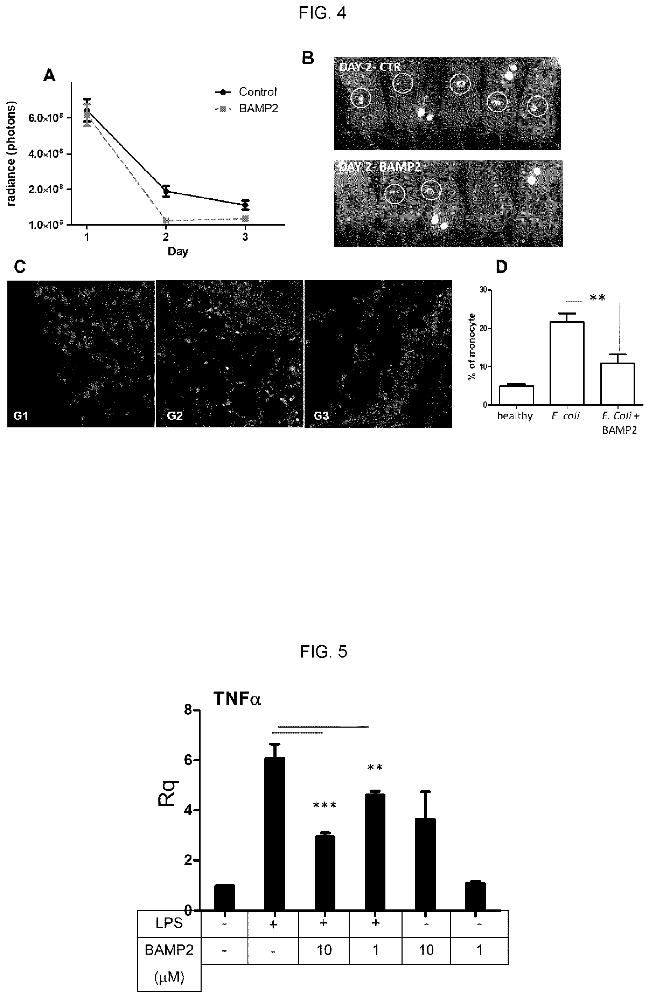

**Fig. 4** Model of an E. coli-luxCDABE skin infection. (A) The number of E. coli cells was measured by radiance with an IVIS Lumina X5 imager. Two groups, treated with BAMP2 and untreated, were compared. An unpaired *t*-test was performed with n=5, 95% confidence interval, p<0.05. (B) Images taken 48 hours after infection of the BAMP2-treated group and the untreated control group (white dots are an artifact of the visible light beam used to take the image, and are not calculated as radiance); the fluorescent signal of the bacteria is highlighted by a white circle. (C) Immunohistochemistry of fixed skin cryosections. The blue signal (DAPI, in b/w light gray) was used to evaluate the total number of cells in the tissue slice sample, and the green signal (anti-Ly6-G/Ly6-C-488, blank in b/w) indicates monocytes. (D) Percentage of monocytes on of the total number of cells. Confocal microscopy images were analyzed with ImageJ (n = 3-6), followed by a two-tailed *t*-test (**, p<0.05).
**Fig. 5** In the presence of BAMP2, cells express a lower amount of TNFα, as measured in a real-time PCR experiment.

### EXPERIMENTAL PART

### Example 1: Peptide selection and synthesis

A small library of eighteen peptides was obtained using sequences of 9-12 natural AMP residues. The sequences were derived from the linear peptides dermaseptin, mastoparan, uperin, defensin, bovine hemoglobin α-chain (α-CBH), Anoplin and cathelicidin, and were selected from the Database of Antimicrobial Activity and Structure of Peptides (DBAASP- https://dbaasp.org/home), filtering them based on their proven efficacy and low toxicity to eukaryotic cells, in linear form.

Branched peptides synthesized in solid phase have optimal yields starting from a length of approximately 15 residues. Longer peptides may tend to aggregate on the resin when their side chains are protected, resulting in poor yields and the formation of numerous byproducts. Ten of the eighteen peptides filtered from the database were already of acceptable length; the others (BAMP37, 39, 41, 43, 44, 45, 46, 49, 51, 52) were split into two fragments. All eighteen peptides (Table 1) were synthesized in a tetrabranched form on an automated multiplex synthesizer using standard Fmoc chemistry. The products were purified and characterized by HPLC and mass spectrometry.

### Antibacterial activity

A first selection cycle of the 18 peptides was carried out against E. coli (Table1).

**TABLE 1**

| **N** | **BAMP** | **Structure** | **Origin** | **MIC (µM) vs** E. coli **TG1** | **R1** |
|---|---|---|---|---|---|
| 1 | 2 | TLLKKVLKAAAK)₄K₂K | Dermaseptin | 1.5 | SEQ ID NO: 1 |
| 2 | 13 | INLKAIAALAKKLL)₄K₂K | Mastoparan | 12.5 | SEQ ID NO: 13 |
| 3 | 18 | GIIDIAKKLFESW)₄K₂K | Uperin | 6.5 | SEQ ID NO: 3 |
| 4 | 21 | LSAAHSLAIGRR)₄K₂K | Defensin | 25 | SEQ ID NO: 14 |
| 5 | 22 | LSAARSLAIGRR)₄K₂K | Defensin | 12.5 | SEQ ID NO: 15 |
| 6 | 23 | LRAAHRLAIGRR)₄K₂K | Defensin | 6 | SEQ ID NO: 4 |
| 7 | 26 | STVLTSKYR)₄K₂K | α-CBH | 25 | SEQ ID NO: 16 |
| 8 | 30 | ALLKRIKTLL)₄K₂K | Anoplin | 25 | SEQ ID NO: 17 |
| 9 | 34 | KLLKFIKTLL)₄K₂K | Anoplin | 3 | SEQ ID NO: 18 |
| 10 | 35 | KLLKRIKKLL)₄K₂K | Anoplin | 1.5 | SEQ ID NO: 5 |
| 11 | 37 | KKLLGKNWKLM)₄K₂K | Mastoparan | 1.5 | SEQ ID NO: 6 |
| 12 | 39 | LKKVLKAAAK)₄K₂K | Dermaseptin | 1.5 | SEQ ID NO: 7 |
| 13 | 41 | LKLKSIVSW)₄K₂K | Mastoparan | 6 | SEQ ID NO: 8 |
| 14 | 45 | INLKKLAKL)₄K₂K | Mastoparan | 1.5 | SEQ ID NO: 9 |
| 15 | 46 | RGLRRLGRKI)₄K₂K | Cathelicidin | 6 | SEQ ID NO: 19 |
| 16 | 49 | GRFKRFRKKL)₄K₂K | Cathelicidin | 1.5 | SEQ ID NO: 10 |
| 17 | 51 | KRLWHKVGPF)₄K₂K | Cathelicidin | 3 | SEQ ID NO: 20 |
| 18 | 52 | RGLRRLGRKIA)₄K₂K | Cathelicidin | 6 | SEQ ID NO: 11 |

The most effective sequences were 12-mer BAMP2, TLLKKVLKAAAK)4K2K, and its 10-mer analog BAMP39 (LKKVLKAAAK)4K2K) derived from dermaseptin. BAMP35 (KLLKRIKKLL)4K2K) is a 10-mer derivative of Anoplin. The 11-mer BAMP37 (KKLLGKNWKLM)4K2K) and 9-mer BAMP45 (INLKKLAKL)4K2K) are derivatives of mastoparan. BAMP49 (GRFKRFRKKL)4K2K) is a 10-mer derivative that mimics the sequence of ChMAP28, a cathelicidin. This set of 6 peptides was tested in a larger panel of species and strains with varying antibiotic susceptibilities. The MICs of the best BAMPs against Gram-positive and Gram-negative bacteria are shown in Table 2.

The most effective sequences were identified: BAMP2, TLLKKVLKAAAK)4K2K, and its shortened analog BAMP39, LKKVLKAAAK)4K2K, derived from Dermaseptin. BAMP35, KLLKRIKKLL)4K2K, is a 10-mer derivative of Anoplin. The 11-mer BAMP37, KKLLGKNWKLM)4K2K and 9-mer BAMP45, INLKKLAKL)4K2K, are derived from mastoparan. BAMP49, GRFKRFRKKL)4K2K, is a 10-mer derivative that mimics the sequence of ChMAP28, a cathelicidin. Additionally, two BAMP2 derivatives were prepared, namelyBAMP2.2, LLKKVLKAAAK)4K2K (R1= SEQ ID NO: 21) and BAMP2.3, LLKKVLLAAAK)4K2K (R1 = SEQ ID NO: 2).

This set of 8 peptides was tested in a panel of species and strains with varying antibiotic susceptibilities. The MICs of the best BAMPs against Gram-positive and Gram-negative bacteria are shown in Table 2.

**TABLE 2 - Minimum inhibitory concentrations (µM)**

| **Name** | **Sequence** | E. coli ***TG1*** | E. coli ***LC711*/*14*** | ***P*. *aeruginosa ATCC 27853*** | ***P. aeruginosa FI-25 (bla VIM-1*)*** |
|---|---|---|---|---|---|
| **BAMP2** | TLLKKVLKAAAK)₄K₂K | 1.5 | 3 | 6 | 12 |
| **BAMP2.2** | LLKKVLKAAAK)₄K₂K | 1.5 | 0.7 | 1.5 | 1.5 |
| **BAMP2.3** | LLKKVLLAAAK)₄K₂K | 1.5 | 0.7 | 1.5 | 1.5 |
| **BAMP35** | KLLKRIKKLL)₄K₂K | 1.5 | 6 | 6 | NT |
| **BAMP37** | KKLLGKNWKLM)₄K₂K | 1.5 | >25 | 3 | NT |
| **BAMP39** | LKKVLKAAAK)₄K₂K | 1.5 | 6 | 6 | NT |
| **BAMP45** | INLKKLAKL)₄K₂K | 1.5 | 6 | 3 | NT |
| **BAMP49** | GRFKRFRKKL)₄K₂K | 1.5 | >25 | 6 | NT |

| **Name** | **Sequence** | ***P. aeruginosa FI-29 (bla GES-5*)*** | ***K. pneumoniae ATCC43816*** | ***K. pneumoniae (colR*)*** |
|---|---|---|---|---|
| **BAMP2** | TLLKKVLKAAAK)₄K₂K | 12 | 6 | 6 |
| **BAMP2.2** | LLKKVLKAAAK)₄K₂K | NT | 3 | NT |
| **BAMP2.3** | LLKKVLLAAAK)₄K₂K | NT | 6 | NT |
| **BAMP35** | KLLKRIKKLL)₄K₂K | NT | 6 | 12,5 |
| **BAMP37** | KKLLGKNWKLM)₄K₂K | NT | 25 | >25 |
| **BAMP39** | LKKVLKAAAK)₄K₂K | NT | 12.5 | >25 |
| **BAMP45** | INLKKLAKL)₄K₂K | NT | 12 | 6 |
| **BAMP49** | GRFKRFRKKL)₄K₂K | NT | 25 | >25 |

| | | | | |
|---|---|---|---|---|
| * bla_{VIM-1}, VIM-1 metallo-β-lactamase; bla _{GES-5}, GES-5 β-lactamase; colistin-resistant phenotype; vanA, vancomycin resistant phenotype; NT: not tested | | | | |

The peptides were incubated at 37°C for 12 hours with the Gram-negative strains E. coli, P. aeruginosa and K. pneumoniae, and the Gram-positive strains *E*. *faecalis,* E. faecium and S. aureus. All showed a more pronounced effect against Gram-negative species. Activity against the reference strain E. coli TG1 ranged from 0.7 to 6 µM. Similarly, the MIC against the clinical isolate E. coli LC711/14 ranged from 3 to 6 µM, except for BAMP37 and BAMP49, which showed lower activity. The reference strain P. aeruginosa was susceptible to all peptides in the 3-6 µM range. BAMP2 showed similar activity (12 µM) against P. aeruginosa clinical isolates FI25 and FI29, which carry two different genes conferring β-lactamase resistance. All BAMPs showed MICs between 6 and 25 µM against the reference strain K. pneumoniae. In recent decades, the emergence of highly drug-resistant Gram-negative strains has led to the renewed use of colistin in clinical practice; consequently, the emergence of resistance due to various acquired mechanisms has been reported. Since colistin and BAMP peptides are thought to have a similar mechanism of action, it is of considerable importance that BAMP2, BAMP35, BAM39, and BAMP45 were active against colistin-resistant E. coli LC711/14, and also against the colistin-resistant K. pneumoniae strain colR, except for BAMP39. Activity against Gram-positive strains *E*. *faecalis* and S. aureus was poor for all peptides, except for BAMP35 against S. aureus USA300, which showed a MIC of 3 µM, and BAMP2 against E. faecium FI-48 with a MIC of 6 µM. Monomeric analogues of the six active sequences were synthesized in linear form and tested against E. coli TG-1 to confirm the gain in efficacy of the branching strategy. For all six peptides, in fact, the MIC was above the maximum concentration tested of 25 µM.

**TABLE 3 - Minimum inhibitory concentrations (µM) of the monomers (µM)**

| Name | Sequence | E. coli TG1 |
|---|---|---|
| MONO2 | TLLKKVLKAAAK (SEQ ID NO: 1) | >25 |
| MONO35 | KLLKRIKKLL (SEQ ID NO: 5) | >25 |
| MONO37 | KKLLGKNWKLM (SEQ ID NO: 6) | >25 |
| MONO39 | LKKVLKAAAK (SEQ ID NO: 17) | >25 |
| MONO45 | INLKKLAKL (SEQ ID NO: 8) | >25 |
| MONO49 | GRFKRFRKKL (SEQ ID NO: 9) | >25 |

### EXAMPLE 2: Cytotoxicity of branched antimicrobial peptides

Red blood cell toxicity is common for antimicrobial peptides (AMPs), and it is often attributed to the slightly negative charge of erythrocytes. This characteristic makes them susceptible to cytotoxic effects of cationic AMPs. To assess hemolysis, a standard colorimetric assay, measuring the absorbance (405 nm) of hemoglobin after 2 hours of incubation at 37°C with various peptide concentrations, was carried out.

BAMP2 showed hemolysis at 5 µM, while its shorter analog, BAMP39, showed no hemolytic activity even at concentrations 10 times higher than the minimum inhibitory concentration (MIC). BAMP35 showed hemolysis at 1.25 µM, indicating greater toxicity at lower concentrations. BAMP45 and BAMP49 induced hemolysis at around 15 µM. Conversely, the mastoparan derivative BAMP37 did not show any hemolytic activity (Figure 1A-F ).

To test toxicity in a eukaryotic model, RAW264.7 murine macrophages were used. Cell viability was measured in a colorimetric assay after 4 and 24 hours of incubation (Figure 1G-H, respectively) with peptides at different concentrations. The slopes were similar for both timepoints, but 100% cell death was observed only at 24 hours. Among the peptides tested, BAMP39 showed the lowest toxicity, requiring a concentration of 200 µM to achieve 100% cell death, with a LC50 of 5,455e-005 M. Conversely, the other peptides, BAMP2, BAMP37, and BAMP49, demonstrated greater toxicity with LC50 values of 4,405e-007 M, 1,000e-005 M, and 1,258e-005 M, respectively. In fact, BAMP2, BAMP37, and BAMP49 induced complete cell death at concentrations 1 log lower than those required for BAMP39 (Fig. 1 G-H)

### EXAMPLE 3: Mechanism of action

Antimicrobial peptides belong to a large family of molecules that share a similar mechanism of action against bacteria. The first step in the killing mechanism is the interaction of the peptides with LPS, deriving from Gram-negative species, or LTA for Gram-positive bacteria. The peptides then act primarily by interacting with bacterial membranes and compromising their function and overall homeostasis, thus leading to cellular distress and eventually to death. The similarities in the killing mechanisms of AMPs are due to typical characteristics of their structure, such as amphipathicity and charge.

The membrane-damaging effect of these peptides against E. coli TG1 was assessed using two fluorophores, propidium iodide and Sytox green. The two dyes are actively fluorescent when binding to nucleic acids, an this occurs only when the cytoplasmic membrane is critically damaged. The membrane-permeability-increasing effect of the peptides was already visible after 10 minutes of incubation for all peptides at twice the MIC. Dose dependence was also apparent for all BAMPs. The increase in fluorescence of the two fluorescent dyes reached a plateau after only 10 minutes at the highest concentrations, and after 40-50 minutes when the peptides were used exactly at the MIC (Fig. 2A-B). Electron microscopy was also used to qualitatively describe the peptides' effect on the membrane of E. coli. Scanning electron microscopy (SEM) images taken after 30 or 60 minutes of incubation at twice the MIC showed membrane deterioration, surface wrinkling, bacterial cell enlargement, and a general loss of surface smoothness, apparent in untreated control bacteria (Fig. 3C). The qualitative effect was similar at both timepoints, but the frequency was higher with longer incubation.

Transmission electron microscopy (TEM) was performed on E. coli following 60-minute treatment with the peptide BAMP2, taken as an arbitrary template for all peptides, and showed loss of integrity of the outer membrane bilayer and formation of vesicles outside the cell (Fig. 2D).

### EXAMPLE 4: Stability to proteolytic activity

In clinical practice, the main obstacle to the development of peptides as antibiotics is their short half-life. Peptides are rapidly degraded by proteases and peptidases; in fact, endogenous peptides of the innate immune system must be continuously produced by cells. Tetrabranched peptides are known to be resistant to proteases because their branched structure hinders access to the binding sites of the proteolytic enzymes. The stability of BAMP peptides in serum, used as a model of a complex mixture of proteases, was tested. The tetrabranched peptides BAMP2, BAMP37, BAMP39, and BAMP49 and their linear analogues MONO-2, MONO-37, MONO-39, and MONO-49 were incubated for 4 hours and 24 hours at 37°C. HPLC analysis, followed by mass spectrometry, confirmed the presence of the intact peptide (Table4).

**TABLE 4. Serum stability of peptides after 4h and 24h incubation at 37°C. Branched peptides are compared with their linear analogues.**

| **Name** | **Sequence** | **Peak presence after 4h** | **Peak presence after 24h** |
|---|---|---|---|
| BAMP2 | TLLKKVLKAAAK)₄K₂K | Yes | Yes |
| MONO-2 | TLLKKVLKAAAK | Yes | No |
| BAM P37 | KKLLGKNWKLM) ₄K₂K | Yes | Yes |
| MONO-37 | KKLLGKNWKLM | No | No |
| BAMP39 | LKKVLKAAAK) ₄K₂K | Yes | Yes |
| MONO-39 | LKKVLKAAAK | No | No |
| BAMP49 | GRFKRFRKKL) ₄K₂K | Yes | No |
| MONO-49 | GRFKRFRKKL | Yes | No |

HPLC and MS peaks were observed after 24 h of incubation for all branched peptides except for BAMP49. All monomeric analogues were completely degraded within 24 h; MONO-37 and MONO-39 were already hydrolyzed at 4 h (Table 4).

### EXAMPLE 5: Binding of BAMPs to LPS

Like many other naturally occurring AMPs, these peptides are rich in cationic amino acid residues with a net positive charge that attracts them towards negatively charged components, such as lipopolysaccharide (LPS) and lipoteic acid (LTA), on the bacterial surface. Typically, the inner core of LPS contains several molecules of KDO (3-deoxy-α-D-manno-octulosonic acid), a negatively charged portion of the macromolecule located very close to the bacterial membrane. The electrostatic interaction between LPS and peptides explains part of the selectivity of AMPs for bacteria rather than eukaryotic cells, and this is therefore very important. Furthermore, the ability to bind LPS is crucial for curbing the onset of inflammation; in this sense, AMPs have been shown to neutralize LPS and prevent its severely toxic effects, released especially during sepsis.

### EXAMPLE 6: Biofilm inhibition activity

Biofilm matrix disruption is critical to bacterial eradication, as it exposes them to treatments. Antimicrobial peptides are known for their ability to penetrate dense, protective bacterial biofilms, presumably due to their amphipathic surfactant nature.

**TABLE 5 - Biofilm inhibitory concentrations (µM). BPC, biofilm prevention concentration; MBIC, minimum biofilm inhibitory concentration.**

| | ***E.coli* TG1** | | | |
|---|---|---|---|---|
| **µM** | **BAMP2** | **BAMP37** | **BAMP39** | **BAMP49** |
| **MIC** | 1.5 | 3 | 1.5 | 6 |
| **BPC** | 1.5 | 3 | 6 | 25 |
| **MBIC** | 3 | 25 | 50 | 50 |

| | ***K. pneumoniae* ATCC 43816** | | | |
|---|---|---|---|---|
| **µM** | **BAMP2** | **BAMP37** | **BAMP39** | **BAMP49** |
| **MIC** | 6 | 25 | 12.5 | >25 |
| **BPC** | 50 | 50 | >50 | 50 |
| **MBIC** | 50 | 50 | 50 | 50 |

Biofilm inhibition activity was assessed based on two parameters: biofilm prevention concentration (BPC) and minimum biofilm inhibition concentration (MBIC). BPC is the lowest peptide concentration that results in 20% biofilm formation with respect to untreated controls. Biofilm formation was measured after removing planktonic cells by washing the wells, previously incubated for 24 hours with bacteria and the peptide. MBIC is the lowest peptide concentration that results in an 80% reduction in preformed biofilm with respect to untreated controls. A 24-hour biofilm was challenged with serial dilutions of the peptides for 14 hours. The residual biofilm was measured after removing the planktonic cells. Crystal violet absorbance at 585 nm was used to measure the biofilm in both cases.

In E. coli, BAMP2 and BAMP37 showed a BPC equal to the MIC; BAMP2 was also efficient in removing preformed biofilm at a concentration twice the MIC (Table 4). For BAMP37, the BPC was eight times the MIC. BAMP39 and BAMP49 showed a MBIC four times the MIC. The MBIC of BAMP39 and BAMP49 was weaker (50 µM). K. pneumoniae biofilm was more resistant to treatment with all BAMPs (Table 5).

### EXAMPLE 7: Efficacy of BAMP2 in In Vivo Infections

Animals were infected with 1 10⁸ CFU of E. coli-luxCDABE subcutaneously on the back and treated locally by s.c. injection of BAMP2 (16 mg/kg) for three consecutive days. E. coli-luxCDABE was obtained by transfecting E. coli TG1 with the pGEN-luxCDABE plasmid, kindly donated by Harry Mobley (plasmide Addgene # 44918; http://n2t.net/addgene:44918; RRID:Addgene_44918). All bacteria were grown at 35°C in their specific medium.

Images of the mice backs were taken daily and recorded as radiance. The BAMP2-treated group showed a 75% reduction in bacterial load compared to the untreated group on day 2, when the maximum difference was noticeable (Fig. 4A). Differences between the treated and untreated groups were calculated based on luminescence of the medium from each group at each time point, and they were significant on days 2 and 3. Luminescence decreased over time in all groups as a result of the mice's immune response and the decline in luciferase expression.

### EXAMPLE 8: BAMP2 In Vivo Immunomodulatory Capabilities

AMPs also have immunomodulatory properties that contribute to the resolution of inflammation, preventing excessive and prolonged inflammatory responses that can lead to tissue damage or shock, as in sepsis. AMPs modulate the immune response by binding lipopolysaccharides and lipoteic acid, and by modulating cytokine production. The anti-inflammatory properties of BAMP2 were tested in the *in vivo* model by measuring the recruitment of inflammatory monocytes to the site of infection using a specific antibody (anti-Ly6-G/Ly6-C) on tissue slices. Healthy skin from uninfected animals showed extremely low monocyte counts, while recruited immune cells were more than 20% higher in infected skin (Fig. 4 C and D). In treated mice, the number of monocytes was reduced to almost that of healthy skin, demonstrating that BAMP2 can significantly impair inflammation.

BAMP2 has also demonstrated anti-inflammatory activity *in vitro.* RAW264 murine macrophage cells were used which, when treated with LPS, release TNFα, an inflammatory mediator. The peptide BAMP2 is able to reduce TNFα expression by LPS-stimulated macrophages (Fig. 5).

### EXAMPLE 9: Bactericidal Activity in a Cream

In order to evaluate the peptides' potential in cream formulations suitable for use as topical antibiotics for skin infections or acne, as well as cosmetic preservatives, a study on the antimicrobial activity of peptides incorporated into the hydrophilic cream base (AIESI) was carried out.

Approximately 10⁸ CFU of *E.coli* TG1 bacteria were plated in a 96-well plate, in 50% cream, 50% MHB. Peptides at various concentrations were added to the mixture. Plates were incubated at 37°C for 18 hours, and turbidity was then measured to assess bacterial growth.

MICs of the peptides in the cream formulation are shown in Table 6

| | ***E.coli* TG1** | |
|---|---|---|
| **µM** | **BAMP2** | **BAMP39** |
| **MIC** | 1.5 | 3 |

Both peptides demonstrated antibacterial efficacy when incorporated into a cream base, at the same active concentrations as in the absence of cream.

### EXAMPLE 10: Bactericidal activity in gel matrices

In order to evaluate the potential of peptides when incorporated into three-dimensional gel-like matrices, a study on the antimicrobial activity of peptides incorporated into collagen was carried out.

The incorporation of antimicrobial and immunomodulatory peptides into collagen, or other materials with gelling properties, can be of fundamental importance for the development of biomaterials for many types of applications, such as the creation of scaffolds for guided bone and/or dental tissues regeneration, and the simultaneous prevention of infections; wound dressings; and antimicrobial coatings for surgical implants.

Approximately 10⁸ CFU of *E.coli* TG1 bacteria were plated in a 96-well plate in 50% collagen type I or type IV (0.78 mg/ml), 50% MHB. Peptides at various concentrations were added to the mixture. Plates were incubated at 37°C for 18 hours, and turbidity was then measured to assess bacterial growth.

MICs of the peptides in the gel formulation are shown in Table 7

| | ***E.coli* TG1** | |
|---|---|---|
| **µM** | **BAMP2** | **BAMP39** |
| **MIC** | 1.5 | 1.5 |

Both peptides showed antibacterial efficacy when incorporated into collagen, at the same active concentrations as in the absence of a gel. Therefore, they hold promises for the development of gels for wound treatment and bone infections in orthopedics or endodontics.

### EXAMPLE 11: Bactericidal activity in endodontic infection models

Endodontic and periodontal diseases are conditions of infectious origin that can lead to teeth loss or develop into systemic hyperinflammation, which can be associated with a wide variety of diseases, including cardiovascular diseases. The use of antimicrobial peptides to irrigate dental cavities is a promising new field of development for fighting and preventing dental infections.

The tetrabranched peptides were tested against bacterial strains typical of root canal infections.

**TABLE 8 MIC (µM)**

| **NAME** | **SEQUENCE** | **E Coli TG1** | ***E*. *faecalis* 51299** | ***E*. *faecalis* post endodontic treatment isolate** | **S. aureus USA 300** |
|---|---|---|---|---|---|
| **BAMP2D** | **tllkkvlkaaak**)₄K₂K | 0.7 | 25 | 25 | 12 |
| **BAMP2.2** | **LLKKVLKAAAK**)₄K₂K | 1.5 | 100 | 100 | NT |
| **BAMP2.3** | **LLKKVLLAAAK)**₄K₂K | 1.5 | 50 | 25 | NT |
| **BAMP39D** | **llkkvlkaaak**)₄K₂K | 25 | 25 | 25 | 25 |

| | | | | | |
|---|---|---|---|---|---|
| NT: not tested. | | | | | |

The two tetrabranched peptides BAMP2D and BAMP39D are analogs of BAMP2 and BAMP39, respectively, but all the amino acids in their sequence are in the D form. They showed activity against the main bacterial species causing endodontic infections, *E. faecalis.* They also showed activity against another Gram-positive species, *S*. *aureus.* Furthermore, the peptide BAMP2.3 showed efficacy against *E*. *faecalis* (Table 8).

These results confirm that these antimicrobial peptides are valid alternatives for dental treatments against infections.

## Claims

1. A branched peptide or pharmaceutically acceptable salts thereof, said peptide having formula (I) 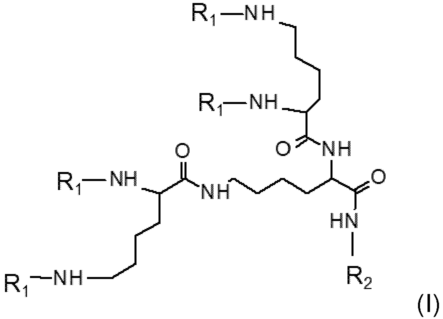 wherein
R₁ equal or different from each other are peptides having SEQ ID NO. 1-12, or portions thereof, or homologs thereof with at least 80% homology, wherein the amino acids can be of the L series or D series;
R₂ is Ala (A), beta-Ala (beta-A), PEG, or is absent.

2. The branched peptide according to claim 1, wherein R₁ are equal to each other.

3. The branched peptide according to any one of claims 1-2, wherein R₁, equal to each other, are selected from the group consisting of SEQ ID NO. 1, SEQ ID NO. 2, and SEQ ID NO. 12.

4. The branched peptide according to any one of claims 1-3, wherein R₂ is absent.

5. A branched peptide according to any one of claims 1-4 for use as a medicament.

6. The branched peptide for use according to claim 5 for use in the treatment of bacterial infections.

7. A pharmaceutical or cosmetic composition comprising a branched peptide according to any one of claims 1-4 and at least one other pharmaceutically acceptable ingredient.

8. The pharmaceutical composition according to claim 7, wherein the administration is by topical route.

9. Use of a branched peptide according to any one of claims 1-4 as a preservative in a cosmetic composition.

10. A gel comprising a branched peptide of formula (I) according to any one of claims 1-4.

11. The gel according to claim 10 based on collagen.

12. The gel according to any one of claims 10-11 for use in the treatment of wounds, or for use in the treatment of bone infections in orthopedics or endodontics.

13. Use of the gel according to any one of claims 10-11 as an antimicrobial coating for prosthetic implants.

14. A wound dressing comprising a gel according to any one of claims 10-11.
